# EUROPEAN PATENT APPLICATION

(11) **EP 4 459 279 A1**
(43) Date of publication of application: **06.11.2024**
(21) Application number: 24172688.4
(22) Date of filing: 26.04.2024
(51) Int. Cl.: G01N 33/02, G01N 21/85, G01N 27/04

(54) **MONITORING DEVICE**

(30) Priority: 04.05.2023 IT 202300008835
(71) Applicant: Autotrasporti Mozzarelli Mauro & C. S.A.S., 46025 Poggio Rusco (MN) (IT); Jack Soft S.r.l., 41012 Carpi (IT)
(72) Inventor: LOSI, Giacomo, Soliera (IT)
(74) Representative: Lunati & Mazzoni S.r.L.

(57) **Abstract**

A monitoring device (1) is provided for a chamber (11) suitable for containing granular material (10), the chamber (11) including a door (110) communicating with the outside environment and comprising closure means (111), the device (1) comprising: sensors (2) capable of detecting physical parameters of the granular material (10); optical instruments (3) capable of recording images of the granular material (10); processing means (4) operationally connected to the sensors (2) and to the optical instruments (3) and configured to process the physical parameters and images, the physical parameters include at least the conductance of said granular material (10), the processing means (4) define algorithms (40) configured to classify the granular material (10) based on the physical parameters and images.

## Description

The present invention relates to a monitoring device of the type specified in the preamble of the first claim.

The object of the present invention is a monitoring device that finds application in the field of storage and transport of materials, particularly granular feeds or edible material.

Monitoring devices that are currently known are used in the transport of granular material. These devices allow for real-time safety checks on goods, typically consisting of granular materials such as, for example, livestock feeds. In particular, technical solutions are known that allow monitoring the transported material parameters using sensors installed on board the transport vehicle. For example, some devices employ sensors that measure in real time the quantity of material being unloaded from the transport vehicle during an unloading operation. In another solution, sensors are used to estimate the weight and weight distribution of the material.

Furthermore, solutions have been developed wherein sensors arranged in different areas of the transport vehicle allow checking which areas are occupied by the transported material and sending a signal when a variation in loading conditions is detected.

More generally, the sensors installed on the transport vehicle allow to visually monitor, and thus assess the state of preservation of the transported material.

In other known solutions, visual control is also performed to determine if the material is properly distributed within the vehicle and that it does not undergo displacements.

These solutions are also coupled with electronic systems that record data relating to loading and unloading operations, such as, for example, the date, time, or place where they were carried out, as well as the quantity of material loaded and unloaded. However, the described known art includes some significant drawbacks.

In particular, the known solutions are largely limited to a real-time inspection of the transported material from a quantitative perspective.

Moreover, the known monitoring devices that perform an optical monitoring function are limited to assessing the variation in loading conditions.

In this situation, the technical task underlying the present invention is to devise a monitoring device capable of substantially overcoming at least part of the previously mentioned drawbacks.

Within this technical task, an important aim of the invention is to obtain a monitoring device that performs a qualitative assessment of granular material as well as quantitative assessment.

Another important aim of the invention is to create a monitoring device that allows assessing the state of preservation for a granular material.

Finally, a further aim of the invention is to obtain a monitoring device that allows automating the control process of a granular material.

The technical task and specified aims are achieved by a monitoring device as claimed in the attached claim 1.

Preferred technical solutions are highlighted in the dependent claims.

The features and advantages of the invention are clarified below by the detailed description of preferred embodiments of the invention, with reference to the accompanying drawings, wherein:

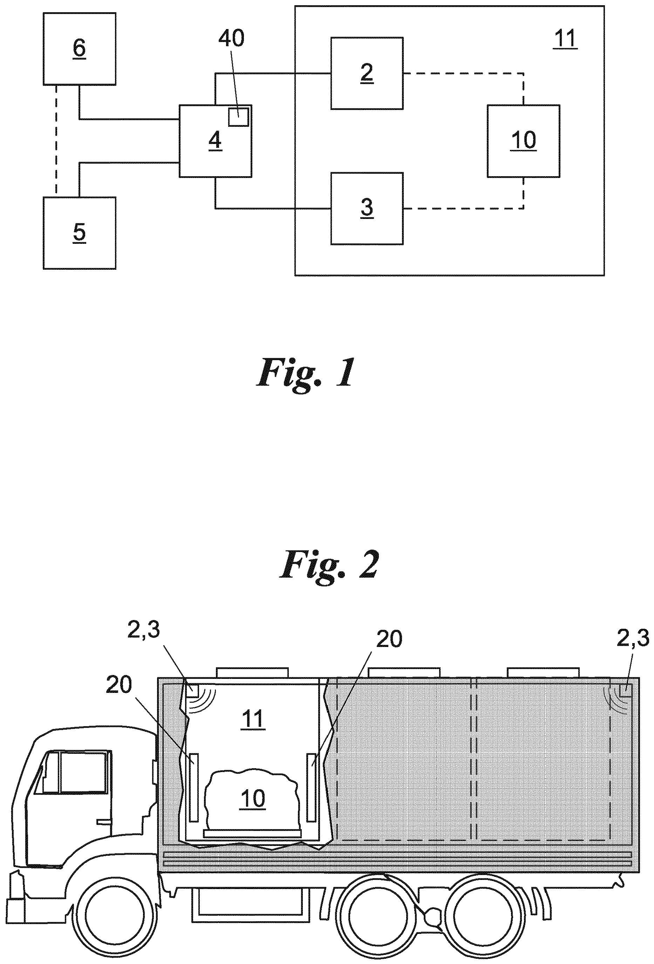

- Fig. 1 shows a schematic operation of a monitoring device according to the invention; and
- Fig. 2 schematically shows a vehicle whereon a monitoring device according to the invention is found.

In this document, measurements, values, shapes, and geometric references (such as perpendicularity and parallelism), when associated with words like "about" or other similar terms such as "approximately" or "substantially," are to be understood as allowing for measurement errors or inaccuracies due to production and/or manufacturing errors and, above all, allowing for a slight deviation from the value, measurement, shape, or geometric reference associated with it. For example, such terms, when associated with a value, preferably indicate a deviation not exceeding 10% of the value itself.

Moreover, when used, terms like "first", "second", "upper", "lower", "primary", and "secondary" do not necessarily identify an order, a priority of relationship, or relative position, but can simply be used to more clearly distinguish different components from one another.

Unless otherwise specified, as it results from the following description, it is considered that terms like "treatment", "computing", "determination", "calculation", or similar, refer to the action and/or processes of a computer or similar electronic computing device that manipulates and/or transforms data represented as physical data such as electronic magnitudes of registers of a computer system and/or memories, other data similarly represented as physical quantities within computer systems, into registers, or other information storage, transmission, or display devices.

Measurements and data reported in this text are to be considered, unless otherwise indicated, as made in International Standard Atmosphere ICAO (ISO 2533:1975). Referring to the Figures, the device according to the invention is globally denoted by the number 1.

The device 1 is a monitoring device for chamber 11 suitable to contain granular material 10.

It is preferably on board a vehicle but can also be on a fixed silo or something else. Chamber 11 may be, for example, a container housed on a truck used for the transport of goods, a fixed silo, or something else. The granular material 10 may be any type of material in granular or powder form. It may be a material for food uses such as, for example, livestock feed.

Chamber 11 includes a door 110 communicating with the outside and including closing means 111. Door 110 preferably defines an adjustable closure. The adjustable closure has the advantage of being able to accordingly adjust the quantity of granular material 10 that can be taken outside of chamber 11.

Device 1 includes sensors 2. Sensors 2 are capable of detecting physical parameters of granular material 10.

Sensors 2 are preferably configured to detect the temperature of chamber 11. Temperature is a parameter advantageous for monitoring operations as it affects the state of preservation of granular material 10. Sensors 2 capable of detecting temperature may include a thermocouple.

Sensors 2 may include a mass detector for granular material 2. In this way, it becomes possible to know the quantity of granular material 10 being inside chamber 11.

In general, the physical parameters detected may be of various types. In particular, they advantageously include at least the conductance of granular material 10.

In this regard, sensors 2 preferably include a pair of plates 20. Plates 20 may be sheets of conductive material, typically a metal with high electrical conductivity. They are preferably separated and electrically isolated from the walls of chamber 11. In this way, chamber 11 is isolated from plates 20 and prevents the passage of electrical current inside chamber 11. Indeed, plates 20 include different electrical potential values. In this way, they can act as an electrical capacitor. Granular material 10 is preferably interposed between plates 20. In this way, plates 20 are electrically connected. In detail, granular material 10 is placed between the two plates 20 in such a way that direct electrical contact is established between the two plates 20 through a conductive path that allows the passage of electric current between a sequence of granules sequentially contacting one anotherand in contact with each of the two plates 20 at the ends.

Sensors 2 are configured to measure at least the electric current, the electrical resistance, and the conductance of granular material 10. For example, sensors 2 may include at least a voltmeter or an ammeter. In this way, the conductance of granular material 10 is measured.

Device 1 includes optical instruments 3. They are capable of recording images of granular material 10. In particular, optical instruments 3 may be cameras. They may be located inside chamber 11. In a possible configuration, cameras may be located in different areas of chamber 11, in such a way as to capture images from multiple angles and also to perform real-time monitoring of the areas occupied by the material inside chamber 11. In any case, the recorded images of granular material 10 allow to obtain information about the material itself.

Therefore, device 1 includes processing means 4. They may include an electronic processing unit. In general, processing means 4 are operationally connected to sensors 2 and optical instruments 3. Therefore, processing means 4 are configured to receive data collected by sensors 2 and by optical instruments 3, namely the physical parameters and images collected. Processing means 4 are therefore configured to process the physical parameters and images.

In particular processing means 4 preferably calculate at least one of a porosity and water content value of granular material 10 starting from its conductance. The values of porosity and water content of granular material 10 allow obtaining information about the state of preservation of granular material 10 and about the type of material.

Moreover, processing means 4 preferably extract at least one of color, shape, and size of granular material 10. This information is obtained from the images collected by optical instruments 3.

Processing means 4 define algorithms 40. They are advantageously configured to classify granular material 10 based on the physical parameters and images. Algorithms 40 are preferably machine learning algorithms optimized to classify granular material 10.

In particular, algorithms 40 are configured to process the physical parameters and images to recognize the type of granular material 10 at the end of the processing. In this regard, machine learning algorithms are optimized to associate each combination of values of physical parameters and images with a type of granular material 10. In detail, the algorithms are optimized to process values of conductance, porosity, water content, shape, color, and size of granular material 10 in order to associate granular material 10 with a material recorded in a database used by the algorithms.

In this way, algorithms 40 perform a classification of granular material 10 based on the collected values of parameters and images. For example, algorithms 40 may associate granular material 10, can distinguish between different types of feed for livestock animals, or they can distinguish between an edible material and a non-edible material.

In this way, device 1 is advantageously able to provide information about the transported material and to perform more rigorous checks; for example, frauds can be prevented when a loading operation with a granular material 10 different from the one to be transported, but of similar appearance, is performed inside chamber 11. Moreover, device 1 allows monitoring the type of load transported by a vehicle or a fixed content. In detail, it preferably includes at least one chamber 11 capable of containing granular material 10 and a device 1.

Device 1 has the advantage of being able to perform real-time classification of granular material 10 transported by each vehicle or silo, in the case of a fleet of vehicles transporting different goods or a set of silos or groups of silos. In this regard, device 1 can be operationally connected to a server 6. Each device 1 present in each vehicle of a fleet can send to server 6 the result of the processing performed by processing means 4 in each vehicle.

Each vehicle can be operationally connected to a satellite global positioning system, such as a GPS, in order to know the position of the vehicle and send it to server 6. Therefore, by combining the result of the processing performed by processing means 4 with the position of the vehicle, it is possible to know in real time the position of a vehicle and what type of load it is transporting.

Algorithms 40 preferably provide information on the state of preservation of granular material 10. This information can be obtained by algorithms 40. Indeed, they can be optimized to also extract this type of information by crossing the values of porosity, water content, conductance with the shape, sizes, and colors obtained from the images.

In this way, it is possible to know if granular material 10 is in good condition and can be transported, or these information can be used to schedule the transport of the load.

In this regard, device 1 can monitor other conditions related to the transport of granular material 10.

In particular, sensors 2 preferably detect the state of closure. Indeed, door 110, defining an adjustable closure, as previously described, can cause the area of the door opening to be adjusted. In some types of unloading operations, for example carried out by tilting chamber 11, the state of the closure of door 110 can be a useful parameter for algorithms 40 to establish the speed at which chamber 11 is emptied. The performance of operations related to loading and unloading can be monitored by device 1.

Indeed, the variation of at least one among the physical parameters, the images, and the state of closure is preferably certified by blockchain.

Therefore, processing means 4 can keep track of the operations performed by updating the blockchain every time a variation of the physical parameters is recorded or every time an operation leading to the variation of the physical parameters and images is performed.

In this way, the transport conditions of granular material 10 are made safer since every operation is monitored and certified, advantageously reducing the risk of fraud or undetected losses of material.

Device 1 may include control means 5. They can be operationally connected to processing means 4. In particular, control means 5 advantageously perform the function of allowing an operator to view the values of the data collected, the images recorded by optical instruments 3, or the results of the processing by processing means 4.

They can be, for example, mobile devices, such as a smartphone or a tablet. Control means 5 can be operationally connected to the satellite global positioning system and to a server 6, so as to monitor the information exchanged between server 6 and device 1.

Control means 5 can also allow the operator to send commands to enable transport operations of granular material 10. For example, control means 5 can command the opening or closing of door 110.

The operation of device 1 previously described in structural terms is as follows. Inside a chamber 11 on board a vehicle, or in a fixed silo, granular material 10 is placed. Inside chamber 11 sensors 2 detect in real-time physical parameters such as porosity, water content, and conductance values of granular material 10. Optical instruments 3, also located inside chamber 11, record the images of granular material 10.

Processing means 4 receive the data collected by sensors 2, namely the porosity, water content, and conductance values. The conductance value can be measured by positioning granular material 10 between two plates 20 there being a potential difference between them. The interposed granular material 10 closes the electrical connection and from the values of electric current and potential difference measured, the conductance of granular material 10 is obtained. Processing means 4, also receive signals from optical instruments 3, that is the shape, colour, and size of the granules of granular material 10. Processing means 4, at this point, process the data using algorithms 40. Algorithms 40 associate a set of data values with a type of granular material 10.

Moreover, algorithms 40 can identify the state of preservation of granular material 10 from the set of values.

Sensors 2 preferably monitor the state of closure of door 110 during the loading and unloading operations of chamber 11.

Moreover, every operation carried out, such as the state of closure of door 110, or a variation of the physical parameters or images is preferably certified by blockchain. Processing means 4 perform the update of the blockchain every time a variation is registered.

An operator can use control means 5 to view the data processed by processing means 4 coming from sensors 2 and from optical instruments 3. Moreover, the operator can output commands to carry out transportation operations of granular material 10.

Device 1 according to the invention achieves significant advantages.

Indeed, device 1 performs a cataloguing of granular material 10 placed inside chamber 11. This cataloguing allows improving the traceability of the transport of goods, as well as certifying their authenticity.

Device 1, therefore, is able to perform both quantitative and qualitative evaluations of granular material 10.

Moreover, device 1 is advantageous because it allows verifying the state of preservation of granular material 10 during transport.

Another advantage of device 1 is to automate the process of controlling granular material 10.

Finally, device 1 allows measuring some physical parameters of granular material 10, such as porosity and water content, by means of a conductance measurement carried out during transport or, in general, when the granular material 10 remains inside chamber 11.

The invention is susceptible to variations that are within the scope of the inventive concept defined by the claims.

Within this scope, all details are replaceable by equivalent elements, and the materials, shapes, and sizes can be any.

## Claims

1. Monitoring device (1) for a chamber (11) suitable to contain granular material (10),
said chamber (11) including a door (110) communicating with the outside environment and comprising closure means (111)
said device (1) comprising:
- sensors (2) capable of detecting physical parameters of said granular material (10);
- optical instruments (3) capable of recording images of said granular material (10);
- processing means (4) operationally connected to said sensors (2) and to said optical instruments (3) and configured to process said physical parameters and said images,
and **characterized in that**
- said physical parameters include at least the conductance of said granular material (10),
- said processing means (4) define algorithms (40) configured to classify said granular material (10) based on said physical parameters and said images.

2. Device (1) according to claim 1, wherein said algorithms (40) are configured to process said physical parameters and said images and to recognize the type of said granular material (10) at the end of said processing.

3. Device (1) according to any of the preceding claims, wherein said processing means (4) calculate at least one among a porosity and water content value of said granular material (10) starting from said conductance.

4. Device (1) according to any of the preceding claims, wherein said sensors (2) include a pair of plates (20) that are separated and electrically isolated from the walls of said chamber (11), said plates (20) comprising different electrical potential values, said granular material (10) being interposed between said plates (20) so as to electrically connect said plates (20), said sensors (2) being configured to measure at least the electric current, electrical resistance, and conductance of said granular material (10).

5. Device (1) according to any preceding claim, wherein said processing means (4) obtain at least one among color, shape, and dimensions of said granular material (10).

6. Device (1) according to any preceding claim, wherein said algorithms (40) provide information on the state of preservation of said granular material (10).

7. Device (1) according to any preceding claim, wherein said sensors (2) are configured to detect the temperature of said chamber (11).

8. Device (1) according to any preceding claim, wherein said door (110) defines an adjustable closure and said sensors (2) detect the state of said closure.

9. Device (1) according to at least claim 8, wherein the variation of at least one among said physical parameters, said images, and said state of said closure is certified by blockchain.

10. Vehicle comprising at least one chamber (11) suitable to contain granular material (10) and a monitoring device (1) for said chamber (11), said chamber (11) and said device (1) being of the type described in any preceding claim.
